Europäisches Patentamt

European Patent Office

Office européen des brevets

(11) Publication number: **0 103 891**
**B1**

## (12) EUROPEAN PATENT SPECIFICATION

(45) Date of publication of patent specification: **26.03.86**

(21) Application number: **83109361.2**

(22) Date of filing: **20.09.83**

(51) Int. Cl.⁴: **C 07 C 69/14, C 07 C 67/293**

(54) Process for producing alpha-acetoxypropionaldehyde.

(30) Priority: **21.09.82 JP 165396/82**

(43) Date of publication of application:
**28.03.84 Bulletin 84/13**

(45) Publication of the grant of the patent:
**26.03.86 Bulletin 86/13**

(84) Designated Contracting States:
**DE FR GB**

(56) References cited:
**FR-A-2 312 485**
**FR-A-2 477 140**

(73) Proprietor: **KURARAY CO., LTD.**
**1621 Sakazu**
**Kurashiki-City Okayama Prefecture (JP)**

(72) Inventor: **Kitamura, Takanori**
**1660, Sakazu**
**Kurashiki City Okayama Prefecture (JP)**
Inventor: **Tamura, Masuhiko**
**961-16, Hirata**
**Kurashiki City Okayama Prefecture (JP)**

(74) Representative: **Schön, Alfred, Dr. et al**
**Patentanwälte Müller-Boré, Deufel, Schön,**
**Hertel Lewald, Otto Isartorplatz 6 Postfach 26 02**
**47**
**D-8000 München 26 (DE)**

Courier Press, Leamington Spa, England.

## Description

### Background of the invention
### Field of the invention

This invention relates to an improved process for producing α-acetoxypropionaldehyde from vinyl acetate.

### Description of the prior art

The present inventors have previously proposed a process for producing α-acetoxy-propionaldehyde from vinyl acetate which comprises hydroformylating vinyl acetate with a mixed gas composed of hydrogen and carbon monoxide in an organic solvent in the presence of a substantially water-insoluble rhodium complex and a trisubstituted phosphine and separating α-acetoxypropionaldehyde from the reaction mixture by extraction with an aqueous medium (cf. DE 31 07 873). According to this proposal, the thermally unstable rhodium catalyst and the reaction product can be separated from each other by extraction with an aqueous medium without exposure to high temperature conditions, and therefore the catalytic activity of the rhodium complex can be maintained stably for a prolonged period of time and the reaction product can be isolated without substantial change in nature. Closer investigations on such a process, however, led to the finding that when the reaction mixture resulting from the hydroformylation reaction carried out under a carbon monoxide partial pressure favorable in selectivity to α-acetoxypropionaldehyde (not lower than about 15 atmospheres (absolute)) is fed as it is to an extraction apparatus for extraction with an aqueous medium, the rhodium complex and trisubstituted phosphine are dissolved in relatively large amounts in the aqueous layer.

### Summary of the invention

Accordingly, an object of the present invention is to provide an improved process for producing α-acetoxypropionaldehyde in an industrially advantageous manner by reducing the loss of rhodium complex and trisubstituted phosphine due to dissolution into the aqueous layer obtained from the extraction with an aqueous medium in the above-mentioned α-acetoxy-propionaldehyde production process.

Other objects of this invention will become apparent as the following description proceeds.

According to the present invention a process is provided for producing α-acetoxypropionaldehyde by hydroformylating vinyl acetate with a mixed gas composed of hydrogen and carbon monoxide in an organic solvent in the presence of a substantially water-insoluble rhodium complex and a trisubstituted phosphine and extracting α-acetoxypropionaldehyde from the reaction mixture with an aqueous medium. This process is characterized in that the reaction mixture is brought into contact with a mixed gas composed of hydrogen and carbon monoxide in a hydrogen/carbon monoxide mole ratio of 1 to 20 with a carbon monoxide partial pressure of 0.2 to 10 atmospheres (absolute) and a hydrogen partial pressure of 1 to 50 atmospheres (absolute) at a temperature of 50° to 100°C prior to the extraction with the aqueous medium.

In accordance with the present invention, α-acetoxypropionaldehyde is formed in high selectivity, the α-acetoxypropionaldehyde is separated from the hydroformylation reaction mixture in good efficiency and the catalytic activity of the rhodium complex is maintained stably for a prolonged period of time, and accordingly α-acetoxypropionaldehyde can be produced in an industrially advantageous manner.

FR—A—2 312 485 relates to a method for the preparation of lactic acid which comprises hydroformylating a vinyl carboxylate with carbon monoxide and hydrogen in contact with a hydroformylation catalyst to prepare an α-acyloxy-propionaldehyde, and oxidizing and hydrolyzing the intermediate to obtain lactic acid. This document discloses that α-acetoxypropionalde-hyde can be prepared by hydroformylation of vinyl acetate in the presence of a rhodium complex and trisubstituted phosphine and that the product α-acetoxypropionaldehyde can be separated from the hydroformylation reaction mixture by distillation. The document neither discloses nor suggests the extraction of the product α-acetoxypropionaldehyde from the hydroformylation reaction mixture as claimed in the present application.

FR—A—2 477 140 and DE—3 107 873 relate to a method for the preparation of α-acetoxy or α-propionyloxypropionaldehyde by hydroformy-lating vinyl acetate or vinyl propionate with a mixed gas composed of hydrogen and carbon monoxide in an organic solvent in the presence of a rhodium complex and trisubstituted phosphine and extracting α-acetoxy- or α-propionyloxy-pro-pionaldehyde from the reaction mixture with an aqueous medium.

The present invention provides an improved process for producing α-acetoxypropionaldehyde in an industrially advantageous manner by reducing the loss of rhodium complex and trisubstituted phosphine due to dissolution into the aqueous layer obtained from the extraction with an aqueous medium in the above-mentioned process. The process of the invention is characterized in that the reaction mixture obtained by the hydroformylation of vinyl acetate is brought into contact with the mixed gas composed of hydrogen and carbon monoxide under such a condition that the hydroformylation reaction may not substantially proceed, prior to the extraction of the product from the hydroformylation reaction mixture with the aqueous medium.

### Detailed description of the invention

The rhodium complex to be used in the present process may be any of the rhodium complexes which are capable of catalyzing the hydroformylation reaction under the reaction conditions and are substantially insoluble in the aqueous medium. A number of such rhodium complexes are known, and such known rhodium complexes are generally usable in the process according to the present invention. Examples are $HRh(CO)(PA_3)_3$, $RhCl(PA_3)_3$, $Rh(acac)_3$, $Rh(OAc)_3$, $Rh_4(CO)_{12}$, $Rh_6(CO)_{16}$, $[Rh(CO)_2(PA_3)_2]_2$, $RhCl_3 \cdot 3H_2O$ and $Rh_2O_3$, wherein A is an aryl group, acac is an acetylacetonato group and OAc is an acetoxy group. Among them, rhodium complexes of the type $HRh(CO)(PA_3)_3$ wherein A is as defined above, are most preferred from the viewpoints of catalytic activity, solubility, ease of handling and so on. The rhodium complex is generally used in the reaction in a rhodium concentration of 0.5 to 5 millimoles per liter of the hydroformylation reaction mixture. The organic solvent used for the practice of the present invention should be substantially water-insoluble solvent in order to facilitate the subsequent extraction of the reaction mixture with an aqueous medium. Many organic solvents qualify in this regard, but when such physical and chemical properties are considered as the solubility of the catalyst components, the loss of the catalyst components due to dissolution into the aqueous layer, price, and possible influences on the subsequent separation step, it is advantageous to employ aromatic hydrocarbons which may optionally be substituted by a lower alkyl group or groups, such as benzene, toluene, xylene and ethylbenzene, and substituted or unsubstituted alicyclic hydrocabrons, such as cyclohexane and methylcyclohexane. The trisubstituted phosphine is represented by the general formula

$$PR'R''R'''$$

wherein R' and R'' each is an aromatic hydrocarbon group and R''' is an aromatic hydrocarbon group or a saturated aliphatic hydrocarbon group containing not less than 3 carbon atoms. Examples of such trisubstituted phosphine are substituted or unsubstituted triarylphosphines, such as triphenylphosphine, tritolylphosphine and trinaphthylphosphine, and diarylalkyl-phosphines, such as diphenylpropylphosphine and diphenylhexylphosphine. Among them, especially preferred are substituted or unsubstituted triarylphosphines. The trisubstituted phosphine is desirably used in an amount of about 5 to about 50 moles per gram atom of rhodium, and more strictly in a concentration of 10 to 100 millimoles per liter of hydroformylation reaction mixture.

In view of the selectivity to α-acetoxypropion-aldehyde, catalyst life and cost of equipment, among others, the hydroformylation of vinyl acetate in accordance with the present invention is preferably carried out at a reaction temperature of 50° to 100°C, a carbon monoxide partial pressure of 15 to 50 atmospheres (absolute), a reaction pressure of 30 to 150 atmospheres (absolute), with hydrogen-to-carbon monoxide mole ratio ($H_2/CO$) in feed gas being 0.5 to 3. The hydroformylation reaction can be carried out either continuously or batchwise in a stirred-tank or bubble-tower reactor. It is advantageous to conduct the reaction while maintaining the vinyl acetate concentration in the reaction zone within a certain range by feeding vinyl acetate continuously. By doing so, heat accumulation during the reaction can be excluded, the selectivity to α-acetoxypropionaldehyde can be increased and the accumulation of high-boiling byproducts can be minimized. From the viewpoints of accumulation of high-boiling byproducts, dissolution of rhodium complex and trisubstituted phosphine into aqueous layer, efficiency of extraction of α-acetoxypropionaldehyde into aqueous layer and so on, the concentration of α-acetoxypropion-aldehyde in the reaction mixture is advantageously maintained within the range of about 0.5 to about 3 moles per liter of reaction mixture.

The reaction mixture containing α-acetoxy-propionaldehyde from the hydroformylation reaction step is, prior to extraction with an aqueous medium, brought into contact with a mixed gas composed of hydrogen and carbon monoxide in a hydrogen/carbon monoxide mole ratio of 1 to 20 with a carbon monoxide partial pressure of 0.2 to 10 atmospheres (absolute) and a hydrogen partial pressure of 1 to 50 atmospheres (absolute) at a temperature of 50° to 100°C. Temperatures below 50°C are unfavorable either because such contacting step is not effective in substantially diminishing the dissolution of the rhodium complex and trisubstituted phosphine or because said contacting step requires a prolonged period of time. Temperatures exceeding 100°C are also undesirable because the reaction product and rhodium complex contained in the reaction mixture are partly deteriorated or decomposed. The effect, which is producible in accordance with the present invention, is no more substantially obtained if the carbon monoxide partial pressure is less than 0.2 atmosphere (absolute) or more than 10 atmospheres (absolute) and/or if the hydrogen partial pressure is less than 1 atmosphere (absolute). If the hydrogen partial pressure exceeds 50 atmospheres (absolute), the formation of byproduct ethyl acetate as a result of hydrogenation of unreacted vinyl acetate remaining in the reaction mixture becomes undesirably significant. When the hydrogen/carbon monoxide mole ratio is less than 1, the above-mentioned contacting step requires a prolonged period of time. When said mole ratio exceeds 20, the formation of byproduct ethyl acetate as a result of hydrogenation of unreacted vinyl acetate unfavorably increases. In this contacting step, the hydroformylation of vinyl acetate does not proceed at a substantial rate.

The above-mentioned contacting is generally carried out under mechanical stirring or gas flow-caused agitation of the reaction mixture. The time for this contacting is generally selected within the range of about 10 to about 90 minutes (in the case of continuous operation, 15 to 120 minutes in terms of average retention time in treatment vessel). This contacting may be carried out either during the period which the hydroformylation reaction mixture is still retained in the reactor or after transfer of said reaction mixture to a separate vessel.

Following the above contacting, the reaction mixture resulting from the hydroformylation of vinyl acetate is subjected to extraction with an aqueous medium, which, upon phase separation, gives an aqueous layer containing α-acetoxypropionaldehyde and an extraction residue containing the catalyst components. The extraction residue can be recycled to the hydroformylation step for reuse. Suitable aqueous extraction media includes water and aqueous solutions containing a small amount of acetic acid. Suitable extraction equipment include any of the known extraction towers of the stirring vessel type, extraction towers of the RDC (rotary disk contractor) type, and perforated plate towers. Close investigations by the present inventors have revealed that the rate of extraction of α-acetoxypropionaldehyde into the aqueous layer and the rate of dissolution of the rhodium complex and trisubstituted phosphine into the aqueous layer depend on the efficiency of contact between the aqueous medium and the hydroformylation reaction mixture, the extraction temperature, the α-acetoxypropionaldehyde concentration in the reaction mixture, the volume ratio between the reaction mixture and the aqueous medium, and the gaseous phase atmosphere during extraction, among others, and that a higher efficiency of contact between the aqueous medium and the hydroformylation reaction mixture, a lower extraction temperature and a higher ratio by volume of the aqeuous medium to the hydroformylation reaction mixture tend to result in a higher rate of extraction of α-acetoxypropionaldehyde into the aqueous layer and reduced losses of the rhodium complex and trisubstituted phosphine by dissolution into the aqueous layer. The extraction temperature is selected within the range of about 5°C to about 40°C. The volume ratio of the aqueous medium to the hydroformylation reaction mixture should desirably be selected depending on the α-acetoxypropionaldehyde concentration in the reaction mixture, for instance within the range of 0.3 to 3 when said concentration is about 0.5 to about 3 moles per liter of the reaction mixture. The extraction is desirably carried out in an atmosphere consisting of a substantially oxygen-free inert gas, such as nitrogen gas, helium gas or argon gas, a hydrogen-carbon monoxide mixture gas having a carbon monoxide partial pressure of at least 0.1 atmosphere (absolute) or such hydrogen-carbon monoxide mixture gas diluted with an inert gas such as mentioned above, whereby the dissolution of the rhodium complex into the aqueous layer can further be suppressed. It is advantageous from the industrial standpoint to perform the extraction in a continuous manner, although it may also be carried out batchwise.

α-Acetoxypropionaldehyde is recovered from the aqueous layer containing the same obtained in the extraction step, by subjecting the aqueous layer to fractional distillation or by subjecting the aqueous layer to extraction with isopropyl acetate, for instance, as the extractant followed by fractional distillation of the thus-separated isopropyl acetate layer, which contains α-acetoxypropionaldehyde.

The following examples and comparative examples illustrate the present invention in more detail.

Comparative example 1

A 6-liter stainless steel stirred-tank pressure reactor connected with a vinyl acetate reservoir, a catalyst solution (extraction residue) reservoir and a 2.4-liter stainless steel continuous extraction column of the Mixco type and provided with a hydrogen and carbon monoxide inlet and a gas outlet was used as the reactor for the continuous hydroformylation of vinyl acetate. The extraction column was connected with a distilled water reservoir, and the gaseous phase in the extraction column consisted of a nitrogen gas atmosphere. The aqueous extract layer containing α-acetoxypropionaldehyde was continuously collected in a reservoir. Constant-flow feed pumps were used in vinyl acetate charging, catalyst solution feeding to the reactor, hydroformylation reaction mixture feeding to the extraction column and distilled water (extractant) charging into the extraction column, respectively. Hydrogen and carbon monoxide were charged into the reactor each at a constant pressure and a constant flow rate through a pressure- and flow rate-adjusting valve. From the top of the reactor, part of the reaction gas was discharged out of the system continuously at a constant rate of flow.

The continuous hydroformylation of vinyl acetate was conducted continuously for 5 days under continuous extraction of α-acetoxypropionaldehyde from the reaction mixture. Two days after the start of reaction, the reaction results and extraction results indicated that the system then had already reached an equilibrium state.

The hydroformylation reaction conditions and water extraction conditions were as follows:

Hydroformylation reaction conditions
Reaction solvent: toluene;
$HRh(CO)(PPh_3)_3$ concentration: 1.75 millimoles/liter;
$PPh_3$ concentration: 35 millimoles/liter;
Reaction mixture volume in reactor: 3.5 liters;
Mean retention time for reaction mixture: 3.5 hrs;
Vinyl acetate feeding rate: 108 ml/hr;
Reaction pressure: 50 atm.;
Hydrogen feeding rate: 42 Nl/hr.;
Carbon monoxide feeding rate: 35 Nl/hr.;
Reaction temperature: 80°C;
Stirring rate: 570 rpm;
Carbon monoxide partial pressure: 23 atm.

Extraction conditions
Continuous extractant phase: water;
Reaction mixture charging rate: 0.97 liter/hr.;
Distilled water charging rate: 0.38 liter/hr.;
Rate of stirring: 270 rpm;
Extraction column inside temperature: 20°C
The reaction results were as follows:
Vinyl acetate conversion: 69%;
Vinyl acetate concentration in reaction mixture: 0.53 mole/liter;
Rate of extraction of α-acetoxypropionaldehyde into aqueous layer: 91%.

The aqueous extract layer, which was obtained at the rate of 0.5 liter/hour, contained the following products:

| Propionaldehyde | 0.22 mole/liter; |
| Acetic acid: | 0.24 mole/liter; |
| α-Acetoxypropionaldehyde: | 1.88 moles/liter; |
| β-Acetoxypropionaldehyde: | 0.15 mole/liter. |

The rhodium catalyst and $PPh_3$ concentrations in the aqueous extract layer, as determined by atomic absorption photometry and colorimetry (assaying on the phosphorus atom basis), were 0.59 ppm (Rh atom basis) and 25 ppm (P atom basis; equivalent to 211 ppm of $PPh_3$), respectively. These data teach that direct extraction treatment of the hydroformylation reaction mixture with water results in relatively great losses of the rhodium catalyst and $PPh_3$ by dissolution into the aqueous layer.

Example 1
The procedure of Comparative Example 1 was followed. After 5 days of reaction, 150 ml of the reaction mixture then obtained (sampled immediately before the extraction column) was charged into a 300-ml stainless steel, magnetic stirrer-equipped autoclave connected with a 300-ml four-necked flask equipped with thermometer, stirrer, liquid-charging inlet, liquid-extracting outlet and gas inlet and outlet. The system was sufficiently purged with hydrogen gas and then the reaction mixture was treated by stirring at 80°C for an hour under the conditions: carbon monoxide partial pressure—1 atmosphere (absolute) and hydrogen partial pressure—9 atmospheres (absolute). Thereafter, the reaction mixture was sent, under making use of the hydrogen-carbon monoxide pressure, to the above four-necked flask, the inside atmosphere of which was then substituted well with a preliminarily prepared hydrogen-carbon monoxide mixed gas ($H_2$/CO mole ratio: 5/1) and maintained at 20°C. Thereafter, the flask was charged with 60 ml of nitrogen gas-purged distilled water and the flask contents were stirred at 600 rpm for 20 minutes and then allowed to stand for 15 minutes. The aqueous layer was carefully taken out of the system by making use of the mixed gas pressure. The Rh and P concentrations in the aqueous layer were determined by the same methods as in Comparative Example 1 and found to be 0.06 ppm and 10 ppm, respectively, as expressed on the Rh atom and P atom basis, respectively. It was thus revealed that the above treatment of the hydroformylation reaction mixture followed by extraction with water results in markedly reduced losses of the Rh catalyst and $PPh_3$ by dissolution into the aqueous extract layer.

A 2-liter extraction vessel equipped with stirrer, reflux condenser and thermometer was charged with 500 ml of the above-obtained aqueous extract layer and 750 ml of isopropyl acetate. The contents were stirred in a nitrogen atmosphere at 70°C for 20 minutes and then allowed to stand. The isopropyl acetate layer which formed upon standing was taken out of the system. This isopropyl acetate extraction was repeated twice more. The isopropyl acetate layers, which amounted to a total of about 2.5 liters, were combinedly subjected to ordinary fractional distillation to give 230 g of α-acetoxypropionaldehyde as a fraction boiling at 54—56°C under 20 mmHg.

Examples 2—6 and comparative examples 2—5
The whole procedure of Example 1 was followed except that the conditions of the hydroformylation reaction mixture treatment were varied. The so-varied conditions of treatment and the amounts of the Rh catalyst and $PPh_3$ dissolved into the aqueous extract layer are shown in correlation with each other in Table 1. The amounts of the Rh catalyst and $PPh_3$ dissolved are given on the Rh and P atom basis, respectively. The carbon monoxide partial pressure and hydrogen partial pressure data are given in terms of atmospheres (absolute).

## TABLE 1

| | Treatment conditions | | | | Dissolution into aqueous layer | | |
| | Temperature (°C) | Carbon monoxide partial pressure | Hydrogen partial pressure | Time(hrs) | Rh(ppm) | P(ppm) | Remarks |
|---|---|---|---|---|---|---|---|
| Example | | | | | | | |
| 2 | 60 | 5 | 45 | 2 | 0.06 | 9 | |
| 3 | 70 | 1 | 9 | 1 | 0.08 | 12 | |
| 4 | 80 | 0.5 | 4.5 | 1 | 0.09 | 13 | |
| 5 | 80 | 2.5 | 7.5 | 1 | 0.07 | 11 | |
| 6 | 80 | 0.7 | 9.3 | 0.5 | 0.08 | 12 | |
| Comparative Example | | | | | | | |
| 2 | 40 | 1 | 4 | 1 | 0.45 | 22 | |
| 3 | 120 | 1 | 1 | 2 | 0.75 | 24 | Rh catalyst activity decreased. |
| 4 | 80 | 1 | 50 | 2 | 0.07 | 10 | Vinyl acetate hydrogenation took place simultaneously. |
| 5 | 80 | 30 | 20 | 1 | 0.43 | 20 | |

## Claims

1. A process for producing α-acetoxypropionaldehyde by hydroformylation vinyl acetate with a mixed gas composed of hydrogen and carbon monoxide in an organic solvent in the presence of a substantially water-in-soluble rhodium complex and a trisubstituted phosphine and extracting α-acetoxypropionaldehyde from the reaction mixture with an aqueous medium, characterized in that said reaction mixture is brought into contact with a mixed gas composed of hydrogen and carbon monoxide in a hydrogen/carbon monoxide mole ratio of 1 to 20 with a carbon monoxide partial pressure of 0.2 to 10 atmospheres (absolute) and a hydrogen partial pressure of 1 to 50 atmospheres (absolute) at a temperature of 50° to 100°C, prior to the extraction with the aqueous medium.

2. A process as defined in Claim 1, wherein the hydroformylation of vinyl acetate is carried out at a reaction temperature of 50° to 100°C, a carbon monoxide partial pressure of 15 to 50 atmospheres (absolute), a reaction pressure of 30 to 150 atmospheres (absolute), with hydrogen/carbon monoxide mole ratio in feed gas being 0.5 to 3.

## Patentansprüche

1. Verfahren zur Herstellung von α-Acetoxypropionaldehyd durch Hydroformylierung von Vinylacetat mit einem gemischten Gas aus Wasserstoff und Kohlenmonoxid in einem organischen Lösungsmittel in Gegenwart eines im wesentlichen wasserunlöslichen Rhodiumkomplexes und eines trisubstituierten Phosphins und Extrahieren von α-Acetoxypropionaldehyd aus der Reaktionsmischung mit einem wäßrigen Medium, dadurch gekennzeichnet, daß die Reaktionsmischung in Kontakt mit einem gemischten Gas aus Wasserstoff und Kohlenmonoxid in einem Wasserstoff/Kohlenmonoxid-Molverhältnis von 1 zu 20 bei einem Kohlenmonoxidpartialdruck von 0,2 bis 10 Atmosphären (absolut) und einem Wasserstoffpartialdruck von 1 bis 50 Atmosphären (absolut) bei einer Temperatur von 50 bis 100°C von der Extraktion mit dem wäßrigen Medium gebracht wird.

2. Verfahren nach Anspruch 1, dadurch gekennzeichnet, daß die Hydroformylierung des Vinylacetats bei einer Reaktionstemperatur von 50 bis 100°C, einem Kohlenmonoxidpartialdruck von 15 bis 50 Atmosphären (absolut), einem Reaktionsdruck von 30 bis 150 Atmosphären (absolut) mit einem Wasserstoff/Kohlenmonoxid-Molverhältnis in dem Beschickungsgas von 0,5 bis 3 durchgeführt wird.

## Revendications

1. Procédé pour produire de l'α-acétoxypropionaldéhyd par hydroformylation de l'acétate de vinyle au moyen d'un mélange gazeux composé d'hydrogène et de monoxyde de carbone dans un solvant organique en présence d'un complexe de rhodium pratiquement insoluble dans l'eau et d'une phosphine trisubstituée et extraction de l'α-acétoxypropionaldéhyde à partir du mélange réactionnel au moyen d'un milieu aqueux, caractérisé en ce que ledit mélange réactionnel est mis en contact avec un mélange gazeux composé d'hydrogène et de monoxyde de carbone dans un rapport molaire hydrogène/monoxyde de carbone de 1 à 20 avec une pression partielle du monoxyde de carbone de 0,2 à 10 atmosphères (absolues) et une pression partielle d'hydrogène de 1 à 50 atmosphères (absolues) à une température de 50° à 100°C, avant l'extraction par le milieu aqueux.

2. Procédé tel que defini à la revendication 1, dans lequel l'hydroformylation de l'acétate de vinyle est effectuée à une température de réaction de 50° à 100°C, une pression partielle de monoxyde de carbone de 15 à 50 atmosphères (absolues), une pression de réaction de 30 à 150 atmosphères (absolues), le rapport molaire hydrogène/monoxyde de carbone dans le gaz d'alimentation étant de 0,5 à 3.